# EUROPEAN PATENT SPECIFICATION

(11) **EP 1 773 216 B1**
(45) Date of publication and mention of the grant of the patent: **03.11.2010**
(21) Application number: 05762684.8
(22) Date of filing: 22.06.2005
(51) Int. Cl.: A61B 17/34, A61M 25/01, A61F 2/06

(54) **APPARATUS FOR SECURING A GRAFT TO A TUNNELER**
VORRICHTUNG ZUR BEFESTIGUNG EINES TRANSPLANTATES AN EINEM TUNNELIERER
APPAREIL POUR FIXER UN GREFFON À UN DISPOSITIF DE FORMATION DE TUNNEL

(30) Priority: 28.06.2004 US 878579
(43) Date of publication of application: 18.04.2007
(73) Proprietor: Maquet Cardiovascular, LLC, San Jose, CA 95134 (US)
(72) Inventor: HENDERSON, Jamie, S., Oakland, NJ 07436 (US); JOECKEL, Warren, A., Wayne, NJ 07470 (US); MEGERMAN, Joseph, Brookline, MA 02445 (US)
(74) Representative: Schwabe - Sandmair - Marx
(86) International application number: PCT/US2005/022223
(87) International publication number: WO 2006/012193

(56) References cited:
- US-A- 3 999 551
- US-A- 4 545 373
- US-A- 5 665 093
- US-A1- 2003 074 007
- US-B1- 6 565 594

## Description

### FIELD OF THE INVENTION

This invention relates generally to surgical instruments, and more particularly to instruments known as "tunnelers," used for subcutaneous placement of arteriovenous grafts for extracorporeal circulation of the blood, arterial bypasses, and the like.

### BACKGROUND OF THE INVENTION

Many tunnelers currently in use utilize sutures or alligator tip clamps to attach the vascular graft to the inner rod of the tunneling devices by which the graft is pulled back through a subcutaneous tunnel made by the tunneler. Typically, graft placement with these devices involves surgical manipulation and tissue trauma which can lead to infection and pain or discomfort, and necessarily longer recovery times. For these reasons, improvements in how the tunneler is attached to the graft are highly desirable.

US 6,565,594 B1 describes a tunnelling device which is implantable in a body and which includes a flexible covering to protect a medical device from contamination during the handling and implantation of the device in the body. The tunnelling device can be connected to a rigid tunneller tip prior to the implantation process in order to facilitate tunnelling, and for the implantation of a graft engagement portion which is configured to engage with the inner surface of the graft. US 5,665,093 describes a surgical implantation apparatus for implanting a surgical device within a human or animal body. US 2003/0074007 A1 describes a graft delivery system for performing a cardiac by-pass procedure using a graft. A combination of catheters and guide devices through the aorta, coronary artery and the thoracic region can be used to accomplish the procedure.

### SUMMARY OF THE INVENTION

An apparatus is provided for securing a graft to a tunneler during implantation of the graft subcutaneously for vascular access according to claim 1. The apparatus includes an elongated member adapted to be tunneled subcutaneously from an entry point at a first location on the skin surface of a patient to an exit point at a second location on the skin surface of the patient. The elongated member includes a proximal end and a distal end. As used herein, the term "proximal end" refers to the end of the apparatus that first enters the patient's body. The apparatus further includes a graft engagement portion at the proximal end of the elongated member. The graft engagement portion includes at least one proximally facing surface adapted to slideably receive, in a direction toward the distal end, the inner surface of a graft. The graft engagement portion further includes at least one radially projecting element adapted to resist movement of the graft, after the graft has been received over the proximally facing surface, in a direction toward the proximal end.

The apparatus further includes a removable pilot tip for attachment to the proximal end of the elongated member. The removable pilot tip is adapted to facilitate tunneling, and the engagement portion is adapted to be removably secured to the pilot tip. Alternatively, the apparatus may include an integrated pilot tip at the proximal end of the elongated member adapted to facilitate tunneling.

Preferred embodiments of the invention are set forth in the dependent claims.

### BRIEF DESCRIPTION OF THE DRAWINGS

Fig. 1A is a modified cross-sectional view of an apparatus for securing a graft to a tunneler which utilizes a removable pilot tip as described herein;
Fig. 1B is the apparatus as illustrated in Fig. 1A with the pilot tip removed, shown engaged within a graft;
Fig. 2 is a side view of another apparatus for securing a graft to a tunneler which includes an integrated tip as described herein;
Fig. 3 is a side view of yet another apparatus for securing a graft to a tunneler shown without its removable pilot tip as described herein;
Fig. 4A is a perspective view of another apparatus for securing a graft to a tunneler shown without its removable pilot tip as described herein;
Fig. 4B is a cross-sectional view of the embodiment illustrated in Fig. 4A;
Fig. 5A is a perspective view of yet another apparatus for securing a graft to a tunneler shown without its removable pilot tip as described herein;
Fig. 5B is a cross-sectional view of the embodiment illustrated in Fig. 5A;
Fig. 6A is a side view of another apparatus for securing a graft to a tunneler which includes a rotatable tip in its pre-engagement position, shown without its removable pilot tip as described herein;
Fig. 6B is an end view of the embodiment illustrated in Fig. 6A, shown within a graft;
Fig. 6C is the apparatus as illustrated in Fig. 6A with the rotatable tip shown in its engagement position;
Fig. 6D is an end view of the apparatus illustrated in Fig. 6C, shown engaged within a graft;
Fig. 7 is a modified cross-sectional view of yet another apparatus for securing a graft to a tunneler shown without its removable pilot tip and shown engaging a graft as described herein;
Fig. 8 is a modified cross-sectional view of another apparatus for securing a graft to a tunneler shown without its removable pilot tip and shown engaged within a graft as described herein; and
Fig. 9 is a modified cross-sectional view of yet another apparatus for securing a graft to a tunneler shown without its removable pilot tip as described herein.

### DETAILED DESCRIPTION OF THE INVENTION

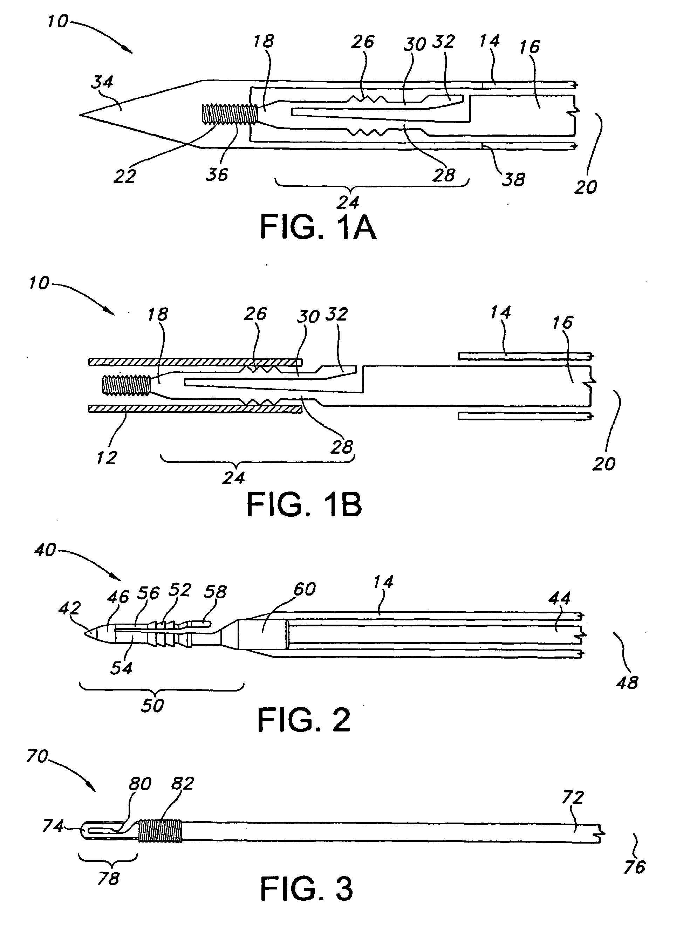

Referring to Fig. 1A, there is shown a sheath tunneling apparatus 10 including a cylindrically-shaped rod-like elongated member 16 which includes a proximal end 18 and a distal end 20. (Distal end 20 is not actually shown in the figure, but is represented as reference numeral 20 for orientation relative to proximal end 18.) Proximal end 18 includes an external thread portion 22, as represented in Fig. 1A. The sheath tunneler 10 includes, among other things, a sheath 14 and a handle (not shown) attached at distal end 20. Apparatus 10 further includes an engagement portion 24 at proximal end 18 of elongated member 16 including radially projecting elements 26. Engagement portion 24 further includes a base portion 28 and a flexible portion 30. A depression member 32 is integrated into flexible portion 30. A removable pilot tip 34 includes an internal thread portion 36 which mates with external thread portion 22 of proximal end 18, thereby attaching removable pilot tip 34 to elongated member 16. Removable pilot tip 34 further includes an abutment surface 38 for retaining sheath 14 along elongated member 16 between removable pilot tip 34 and the handle of the sheath tunneler (not shown).

Flexible portion 30 of engagement portion 24 is biased toward an expanded position as is illustrated in Figs. 1A and 1B. When force is applied to depression member 32 to move flexible portion 30 toward base portion 28, engagement portion 24 is compressed inwardly, reducing its outer diameter at the location containing radially projecting elements 26. (This compressed position is not shown in the figures.)

Elongated member 16, along with sheath 14, is adapted to be tunneled subcutaneously from an entry point at a first location on the skin surface of a patient to an exit point at a second location on the skin surface of the patient. Removable pilot tip 34 is adapted to facilitate tunneling. In use, apparatus 10, including sheath 14, is forcefully passed through the subcutaneous tissue horizontally between two surgically prepared incisions until removable pilot tip 34 is exposed at the second incision or exit wound. Removable pilot tip 34 is then removed from proximal end 18 of elongated member 16 by unscrewing internal threads 36 from external threads 22.

As illustrated in Fig. 1B, engagement portion 24 is adapted to slideably receive, in a direction toward distal end 20, the inner surface of graft 12 over at least one radially projecting element 26 of engagement portion 24. In other words, depression member 32 is depressed (not shown) to compress engagement member 24 and reduce its outer diameter at the location containing radially projecting elements 26. Due to the reduced diameter, graft 12 may then be easily slid over proximal end 18 of elongated member 16 and radially projecting elements 26 of engagement portion 24. Depression member 32 is then released, permitting it to return to its expanded position (as illustrated in Fig. 1B), thereby increasing the outer diameter of engagement member 24 at the location containing radially projecting elements 26.

Radially projecting elements 26 engage the inner surface of graft 12 and are adapted to resist movement of graft 12 in a direction toward proximal end 18. Furthermore, radially projecting elements 26 are adapted to apply outward distension with respect to graft 12. In other words, radially projecting elements 26 provide a secure fit between engagement member 24 and graft 12 by slightly stretching graft 12 outwardly. This secure fit prevents graft 12 from slipping toward proximal end 18 when elongated element 16, with graft 12 attached, is pulled back through the tunnel previously made by tip 34 and its attached components, beneath the skin, and into the implant position. Radially projecting elements 26 are illustrated in Figs. 1A and 1B as V-shaped. The present invention, however, is not limited to V-shaped radially projecting elements. For example, radially projecting elements may be barb-shaped, hook-shaped, or any other shape suitable for achieving the desired engagement.

As indicated above, after attachment of graft 12 to engagement portion 24, elongated member 16 is pulled back toward the entrance wound utilizing the handle (not shown), thereby drawing graft 12 through the entire length of sheath 16 (still positioned within the subcutaneous passageway).

With graft 12 positioned in place, material of graft 12 is then cut free from engagement portion 24 and sheath 14 is carefully extracted from the tissue tunnel and exit wound without extracting graft 12 from the subcutaneous passageway.

For clarity purposes, sheath 14 is represented in Figs. 1A, 1B, 2, and 9 only. However, it is contemplated that each embodiment illustrated in Figs. 3 - 8 is for use with a sheath tunneler. The present invention, however, is not limited to use with a sheath tunneler, and may also be used with a sheathless tunneler.

Fig. 2 illustrates an alternative exemplary configuration of an apparatus 40 for securing a graft (not shown) to a sheath tunneler. A notable difference from the assembly shown in Figs. 1A and 1B, however, is that apparatus 40 includes an integrated pilot tip 42 at proximal end 46 of elongated member 44, thereby eliminating the need for a removable pilot tip.

As in the assembly of Figs. 1A and 1B, apparatus 40 includes a cylindrically-shaped rod-like elongated member 44 which includes a proximal end 46 and a distal end 48. (Distal end 48 is not actually shown in the figure, but is represented as reference numeral 48 for orientation relative to proximal end 46.) Apparatus 40 further includes an engagement portion 50 at proximal end 46 of elongated member 44 including radially projecting barb-shaped elements 52. Engagement portion 50 further includes a base portion 54 and a flexible portion 56. A depression member 58 is integrated into flexible portion 56. Elongated member 44 includes an intermediate portion 60 for retaining sheath 14 along elongated member 44 between engagement portion 50 and the handle of sheath tunneler 40. More specifically, a small clearance fit between the outer diameter of intermediate portion 60 and the inner diameter of sheath 14 adequately secures sheath 14 in place. As illustrated, sheath 14 is optionally tapered at its proximal end to facilitate tunneling.

Similar to the assembly of Figs. 1A and 1B, flexible portion 56 of engagement portion 50 is biased toward an expanded position as is illustrated in Fig. 2. Force may be applied to depression member 58 to move flexible portion 56 toward base portion 54, thereby compressing engagement portion 50 and reducing its outer diameter at the location containing radially projecting barb-shaped elements 52. (This compressed position is not shown in the figure.)

As in the assembly of Figs. 1A and 1B, elongated member 44 is adapted to be tunneled subcutaneously from an entry point at a first location on the skin surface of a patient to an exit point at a second location on the skin surface of the patient. However, the shape of integrated pilot tip 42 illustrated in Fig. 2 is adapted to facilitate tunneling without the use of a removable pilot tip.

The operation of apparatus 40 is virtually the same as that previously described herein with reference to apparatus 10 of Figs. 1A and 1B, without the use of a removable pilot tip. Briefly, apparatus 40 is used to dissect a tunnel by forcing integrated pilot tip 42 through the subcutaneous tissue until integrated pilot tip 42 is exposed. Through manipulation of depression member 58, integrated pilot tip 42 is adapted to slideably receive, in a direction toward distal end 48, the inner surface of the graft over at least one radially projecting barb-shaped element 52 of engagement portion 50, while radially projecting barb-shaped elements 52 are adapted to resist movement of the graft in a direction toward proximal end 46. After attachment of the graft to engagement portion 50, the graft is drawn through the entire length of sheath 14, material of the graft is cut free from engagement portion 50, and sheath 14 is carefully extracted from the tissue tunnel and exit wound without extracting the graft from the subcutaneous passageway.

Fig. 3 illustrates another alternative exemplary configuration of an apparatus 70 for securing a graft (not shown) to a sheath tunneler (not shown). Similar to the assembly of Figs. 1A and 1B, apparatus 70 requires the use of a removable pilot tip (not shown) at proximal end 74 of elongated member 72.

For clarity purposes, a removable pilot tip is not represented in Figs. 3 - 9. Furthermore, the abutment surface of the removable pilot tip (for retaining the sheath along the elongated member), as represented in Fig. 1A as abutment surface 38, is not represented in Figs. 3-9. However, it is contemplated that each embodiment illustrated in Figs. 1A, 1B, and 3 - 9 is for use with a removable pilot tip and includes these features.

As in the assembly of Figs. 1A and 1B, apparatus 70 of Fig. 3 includes a cylindrically-shaped rod-like elongated member 72 which includes a proximal end 74 and a distal end 76. (Distal end 76 is not actually shown in the figure, but is represented as reference numeral 76 for orientation relative to proximal end 74.) Apparatus 70 further includes a hook-shaped engagement portion 78 at proximal end 74 of elongated member 72 including a radially projecting element 80. Elongated member 72 includes a threaded intermediate portion 82 that engages internal threads of a removable pilot tip (not shown) to facilitate tunneling.

As in the assembly of Figs. 1A and 1B, elongated member 72 is adapted to be tunneled subcutaneously from an entry point at a first location on the skin surface of a patient to an exit point at a second location on the skin surface of the patient utilizing a removable pilot tip (not shown) to facilitate tunneling.

The operation of apparatus 70 is similar to that previously described herein with reference to apparatus 10 of Figs. 1A and 1B. Briefly, apparatus 70 is used to dissect a tunnel by forcing a removable pilot tip (not shown) through the subcutaneous tissue until the removable pilot tip is exposed. The removable pilot tip is then removed from proximal end 74 of elongated member 72 by unscrewing internal threads (not shown) from external threads 82.

Engagement portion 78 is adapted to slideably receive, in a direction toward distal end 76, the inner surface of the graft, while radially projecting element 80 is adapted to resist movement of the graft in a direction toward proximal end 74. More specifically, the graft may include a hole in its wall for receiving radially projecting element 80. In other words, radially projecting element 80 may be hooked into a pre-existing hole in the graft wall. Alternatively, the graft may include a loop at its end for receiving radially projecting element 80. Furthermore, a portion of the graft wall at the underside of engagement portion 78 may be cut at an angle to facilitate its entry into the sheath.

After attachment of the graft to engagement portion 78, the graft is drawn through the entire length of the sheath, material of the graft is cut free from engagement portion 78, and the sheath is carefully extracted from the tissue tunnel and exit wound without extracting the graft from the subcutaneous passageway.

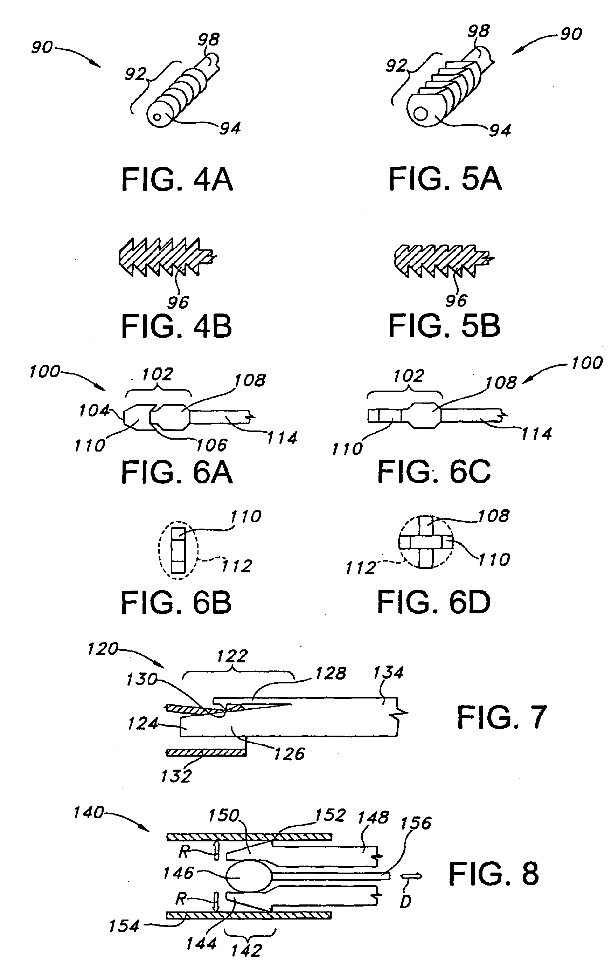

Figs. 4A and 4B illustrate yet another alternative exemplary configuration of an apparatus 90 for securing a graft (not shown) to a sheath tunneler (not shown). Similar to the assembly of Figs. 1A and 1B, apparatus 90 requires the use of a removable pilot tip (not shown) at proximal end 94 of elongated member 98.

For clarity purposes, a threaded intermediate portion (for engagement with internal threads of a removable pilot tip) is not represented in Figs. 4A - 8. However, it is contemplated that each embodiment illustrated in Figs. 3-9 includes this feature.

The portion of the apparatus illustrated in Figs. 4A and 4B includes an engagement portion 92 at a proximal end 94 of an elongated member 98. Engagement portion 92 includes radially projecting barb-shaped elements 96. Engagement portion 92 is adapted to slideably receive, in a direction toward the distal end (not shown), the inner surface of the graft, while radially projecting barb-shaped elements 96 are adapted to resist movement of the graft in a direction toward proximal end 94.

The operation of the apparatus represented in Figs. 4A and 4B is virtually the same as that previously described herein with reference to apparatus 70 of Fig. 3, with an alternative way of attaching the graft to engagement portion 92. More specifically, due to the shape of radially projecting barb-shaped elements 96, the graft (not shown) may be easily slid over proximal end 94 of elongated member 98 and radially projecting barb-shaped elements 96 of engagement portion 92. This is facilitated by the outwardly tapered cross-section of radially projecting barb-shaped elements 96 with each element including a smaller diameter at its proximal and end expanding in a sloped manner to a larger diameter at its distal end. Radially projecting barb-shaped elements 96 engage the inner surface of the graft, and are adapted to resist movement of the graft in a direction toward proximal end 94 by virtue of their circumferential edge and their non-sloped or flat shape distal of that edge. In other words, the circumferential pointed tip of the distally facing flat surface resists proximal movement of the graft relative to elongated member 98. Furthermore, radially projecting barb-shaped elements 96 are adapted to apply outward distension with respect to the graft. This provides a secure fit which prevents the graft from slipping toward proximal end 94 when the graft is pulled beneath the skin and into the implant position.

Figs. 5A and 5B illustrate another alternative exemplary configuration of an apparatus 90 for securing a graft (not shown) to a sheath tunneler (not shown). This embodiment demonstrates an alternative cross section for engagement portion 92, however, the description and operation of this embodiment is virtually the same as that previously described herein with reference to Figs. 4A and 4B. The present invention, however, is not limited to the cross-sectional shapes illustrated in Figs. 4A - 5B. For example, the engagement portion may have a square cross section, a rectangular cross section, a triangular cross section, or any other cross section suitable for achieving the desired graft attachment.

Figs. 6A - 6D illustrate yet another alternative exemplary configuration of an apparatus 100 for securing a graft 112 to an elongated element 114 of a sheath tunneler apparatus (not otherwise shown). The portion of the apparatus 100 illustrated in Figs. 6A - 6D includes an engagement portion 102 at a proximal end 104 of an elongated member 114. Engagement portion 102 includes rotatable radially projecting barb-shaped elements 106. Engagement portion 102 has a rectangular cross section and includes a base portion 108 and a rotatable portion 110 which rotates 90° with respect to base portion 108. Engagement portion 102 is adapted, by virtue of its proximal tapered cross-section, to slideably receive, in a direction toward the distal end (not shown), the inner surface of graft 112, while rotatable radially projecting barb-shaped elements 106 are adapted to resist, by virtue of distally projecting tips, movement of graft 112 in a direction toward proximal end 104.

The operation of the apparatus represented in Figs. 6A - 6D is virtually the same as that previously described herein with reference to apparatus 70 of Fig. 3, with an alternative way of attaching graft 112 to engagement portion 102. More specifically, due to the orientation of rotatable radially projecting barb-shaped elements 106 represented in Figs. 6A (side view) and 6B (end view), graft 112 may be easily slid over proximal end 104 of the elongated member 114. As illustrated in Fig. 6B, graft 112 is loosely disposed about engagement portion 102. Rotatable portion 110 is then rotated 90° with respect to base portion 108, as illustrated in Figs. 6C (side view) and 6D (end view) and rotatable radially projecting barb-shaped elements 106 engage the inner surface of graft 112. As indicated above, rotatable radially projecting barb-shaped elements 106 are adapted to resist movement of graft 112 in a direction toward proximal end 104. Furthermore, rotatable radially projecting barb-shaped elements 106 are adapted to apply outward distension with respect to graft 112, as illustrated in Fig. 6D. In other words, with rotation of portion 110, graft 112 becomes taut, as opposed to its loose condition represented in Fig. 6B. The secure fit prevents graft 112 from slipping toward proximal end 104 when graft 112 is pulled beneath the skin and into the implant position.

Although the apparatus represented in Figs. 6A - 6D is illustrated with rotatable radially projecting barb-shaped elements 106, such barb-shaped elements 106 are optional. In other words, it is contemplated that rotatable portion 110 may sufficiently engage the inner surface of graft 112 without barb-shaped elements when rotated within graft 112 90° with respect to base portion 108. In this optional configuration without barb-shaped elements, rotatable portion 110 is adapted to apply outward distension with respect to graft 112, causing graft 112 to become taut. Similar to the apparatus represented in Figs. 6A - 6D including barb-shaped elements 106, the secure fit of the optional configuration without barb-shaped elements prevents graft 112 from slipping toward proximal end 104 when graft 112 is pulled beneath the skin and into the implant position.

Fig. 7 illustrates another alternative exemplary configuration of an apparatus 120 for securing a graft 132 to a sheath tunneler (not shown). The portion of the apparatus illustrated in Fig. 7 includes an engagement portion 122 at a proximal end 124 of an elongated member 134. Engagement portion 102 includes a base portion 126 and a grasping portion 128. Grasping portion 128 includes a radially inwardly projecting barb-shaped catch 130 configured to engage a wall of graft 132 between grasping portion 128 and base portion 126.

Base portion 126 of engagement portion 122 is adapted to slideably receive, in a direction toward the distal end (not shown), the inner surface of graft 132, while barb-shaped catch 130 is adapted to resist movement of graft 132 in a direction toward proximal end 124.

The operation of the apparatus represented in Fig. 7 is virtually the same as that previously described herein with reference to apparatus 70 of Fig. 3, with an alternative way of attaching graft 132 to engagement portion 122. More specifically, due to the orientation of barb-shaped catch 130, graft 132 may be easily slid over base portion 126 of engagement portion 122. Barb-shaped catch 130 engages the outer surface of graft 132, and is adapted to resist movement of graft 132 in a direction toward proximal end 124. The secure fit prevents graft 132 from slipping toward proximal end 124 when graft 132 is pulled beneath the skin and into the implant position.

Fig. 8 illustrates yet another alternative exemplary configuration of an apparatus 140 for securing a graft 154 to a sheath tunneler. The portion of the tunneler illustrated in Fig. 8 includes an engagement portion 142 at a proximal end 144 of an elongated member 148. Engagement portion 142 includes an actuator 146 attached to a pull element 156 and a flexible collet-type grasping portion 150 disposed about actuator 146. Flexible collet-type grasping portion 150 includes outwardly radially projecting barb-shaped catches 152 configured to engage inner surfaces of graft 154.

Engagement portion 142 is represented in its actuated position in Fig. 8. In its unactuated position (not shown), actuator 146 is positioned toward the left with reference to Fig. 8, thereby permitting flexible collet-type grasping portion 150 (which is biased radially inwardly) to compress, resulting in a smaller outer diameter at the location containing barb-shaped catches 152. Flexible collet-type grasping portion 150 expands upon movement of actuator 146 toward the distal end (not shown) of elongated member 148, i.e., when pull element 156 is pulled in direction D, causing barb-shaped catches 152 to expand outwardly radially (in direction R, as represented in Fig. 8).

Engagement portion 142 is adapted to slideably receive, in a direction toward the distal end, the inner surface of graft 154, while barb-shaped catches 152 are adapted to resist movement of graft 154 in a direction toward proximal end 144.

The operation of the apparatus represented in Fig. 8 is virtually the same as that previously described herein with reference to apparatus 70 of Fig. 3, with an alternative way of attaching graft 154 to engagement portion 142. More specifically, due to the reduced diameter associated with the unactuated position (not shown) of engagement portion 142, graft 154 may be easily slid over proximal end 144 of engagement portion 142 and barb-shaped catches 152 of engagement portion 142. Actuator 146 is then moved toward the distal end (not shown) of elongated member 148, i.e., pull element 156 is pulled in direction D, causing barb-shaped catches 152 to expand radially in direction R, as represented in Fig. 8. Barb-shaped catches 152 engage the inner surface of graft 154, and are adapted to resist movement of graft 154 in a direction toward proximal end 144. Furthermore, barb-shaped catches 152 are adapted to apply outward distension with respect to graft 154. The secure fit prevents graft 154 from slipping toward proximal end 144 when graft 154 is pulled beneath the skin and into the implant position.

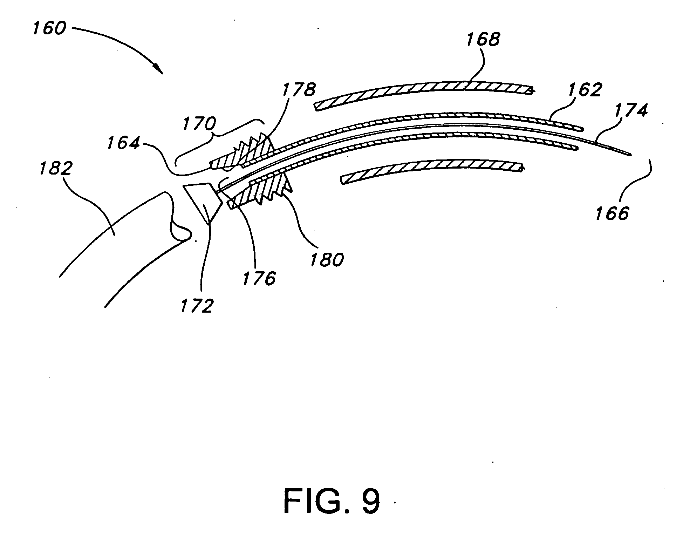

Fig. 9 illustrates another alternative exemplary configuration of an apparatus 160 for securing a graft 182 to a sheath tunneler. Apparatus 160 includes a cylindrically-shaped hollow rod-like elongated member 162 which includes a proximal end 164 and a distal end 166. (Distal end 166 is not actually shown in the figure, but is represented as reference numeral 166 for orientation relative to proximal end 164.) The sheath tunneler includes, among other things, a sheath 168 and a handle (not shown) attached at distal end 166. Apparatus 160 further includes an engagement portion 170 at proximal end 164 of elongated member 162. Engagement portion 170 includes a conical-shaped actuator 172 attached to a pull element 174 (which may be secured at distal end 166 to the handle via a setscrew or camlock mechanism, (not shown)), a conical-shaped opening 176 for receiving conical-shaped actuator 172, and radially projecting elements 178. Engagement portion 170 also includes a threaded intermediate portion 180 that engages internal threads of a removable pilot tip (not shown) to facilitate tunneling.

Engagement portion 170 is adapted to slideably receive, in a direction toward distal end 166, graft 182, while the inwardly radially projecting face 178, in conjunction with conical-shaped opening 176 and mating actuator 172, is adapted to resist movement of graft 182 in a direction toward proximal end 164.

The operation of apparatus 160 represented in Fig. 9 is virtually the same as that previously described herein with reference to apparatus 70 of Fig. 3, with an alternative way of attaching graft 182 to engagement portion 170.

Engagement portion 170 is represented in its unactuated position in Fig. 9. In this position, graft 182 may be easily placed over conical-shaped actuator 172 and, piloted by the conical inner face 178 of opening 176, tucked between conical-shaped actuator 172 and conical-shaped opening 176. Engagement portion 170 may then be actuated (not shown). More specifically, conical-shaped actuator 172 is moved toward distal end 166 of the elongated member 162, i.e., pull element 174 is pulled toward distal end 166, causing conical-shaped actuator 172 to engage tightly within the inwardly projecting face 178 of conical-shaped opening 176, thereby trapping material of graft 182 between conical-shaped actuator 172 and conical-shaped opening 176. In other words, graft 182 is mechanically engaged between conical-shaped actuator 172 and the inwardly projecting face 178 of conical-shaped opening 176 upon movement of conical-shaped actuator 172 toward conical-shaped opening 176. Such mechanical engagement resists movement of graft 182 in a direction toward proximal end 164. The secure fit prevents graft 182 from slipping toward proximal end 164 when graft 182 is pulled beneath the skin and into the implant position.

An exemplary material for forming all apparatus components described herein, namely elongated member, engagement portion, radially projecting elements, integrated pilot tip, removable pilot tip, and pull element is stainless steel. The present invention, however, is not limited to this material, and may include any materials, including, for example, metallic (titanium, for example) or non-metallic (a polymer or other composite material, for example) material that offer desired properties including both strength and flexibility.

## Claims

1. An apparatus for securing a graft to a tunneller while subcutaneously implanting the graft for vascular access, said apparatus comprising:
an elongated member (16, 44, 72, 98, 114, 134, 148, 162) which is designed to be tunnelled subcutaneously from an entry point at a first location on the surface of a patient's skin to an exit point at a second location on the surface of the patient's skin and comprises a proximal end (18, 42, 74, 94, 104, 124, 144, 164) and a distal end (20, 48, 76, 166);
a graft engagement portion (24, 50, 78, 92, 102, 122, 142, 170) at the proximal end (18, 42, 74, 94, 104, 124, 144, 164) of the elongated member (16, 44, 72, 98, 114, 134, 148, 162), wherein the graft engagement portion (24, 50, 78, 92, 102, 122, 142, 170) includes: at least one proximally facing surface which is designed to receive the inner surface of a graft (12, 112, 132, 154, 182) such that it can be slid in the direction of the distal end (20, 48, 76, 166); and at least one radially projecting element (26, 80, 96, 106, 130, 152, 178) which is designed to resist a movement of the graft (12, 112, 132, 154, 182) in the direction of the proximal end (18, 42, 74, 94, 104, 124, 144, 164), once the graft (12, 112, 132, 154, 182) has been received over the proximally facing surface, and a removable pilot tip (34) for attaching to the proximal end (18, 42, 74, 94,104, 124, 144, 164) of the elongated member (16,44, 72, 98, 114, 134, 148, 162), wherein the removable pilot tip (34) is designed to facilitate tunnelling and the engagement portion (24, 50, 78, 92, 102, 122, 142, 170) is designed to be removably secured to the pilot tip (34),
**characterised in that**
the at least one radially projecting element includes a member (32, 58, 80) which is biased radially outwards and comprises at least one barb (26, 80, 96, 106, 130, 152, 178) which projects radially outwards and is configured to engage with the inner surface of the graft (12,112,132, 154, 182).

2. The apparatus according to claim 1, further comprising an integrated pilot tip at the proximal end (18, 42, 74, 94, 104, 124, 144, 164) of the elongated member (16, 44, 72, 98, 114,134,148,162), which is designed to facilitate tunnelling.

3. The apparatus according to any one of the preceding claims, wherein the outwardly biased member (32, 58, 80) is also configured to be temporarily compressed radially, wherein the proximally facing surface of the graft engagement portion (24, 50, 78, 92, 102, 122, 142, 170) at least partially comprises the proximal surface of the inwardly compressible, outwardly biased member (32, 58, 80) which is associated with the engagement portion (24, 50, 78, 92, 102, 122, 142, 170).

4. The apparatus according to any one of the preceding claims, wherein the engagement portion (24, 50, 78, 92, 102, 122, 142, 170) is hook-shaped.

5. The apparatus according to any one of the preceding claims, wherein the engagement portion (24, 50, 78, 92, 102, 122, 142, 170) has a substantially circular cross-section and comprises continuously circumferential barbs which are configured to engage with the inner surface of the graft (12, 112, 132, 154, 182).

6. The apparatus according to any one of the preceding claims, wherein the engagement portion (24, 50, 78, 92, 102, 122, 142, 170) has a substantially circular cross-section and comprises partially circumferential barbs which are configured to engage with the inner surface of the graft (12, 112, 132, 154, 182).

7. The apparatus according to any one of the preceding claims, wherein the engagement portion (24, 50, 78, 92, 102, 122, 142, 170) has a substantially rectangular cross-section and comprises rotatable barbs which are configured to engage with the inner surface of the graft (12, 112, 132, 154, 182) in a first rotatable position and to align with the proximally facing surface in a second rotatable position.

8. The apparatus according to any one of the preceding claims, wherein a part of the engagement portion (24, 50, 78, 92, 102, 122, 142, 170) comprises a base portion and a grasping portion, wherein the grasping portion comprises a catch which is configured to engage with a wall of the graft (12, 112, 132, 154, 182) between the grasping portion and the base portion.

9. The apparatus according to any one of the preceding claims, wherein the elongated member (16, 44, 72, 98, 114, 134, 148, 162) comprises an actuator and a flexible grasping portion which is disposed about the actuator, wherein the flexible grasping portion comprises catches and expands when the actuator is moved toward the distal end of the elongated member (16, 44, 72, 98, 114, 134, 148, 162), wherein the catches are configured to engage with the inner surface of the graft (12, 112, 132, 154, 182).

10. The apparatus according to any one of the preceding claims, wherein the radially projecting element (26, 80, 96, 106, 130, 152, 178) is designed to resist a movement of the graft (12, 112, 132, 154, 182) in the direction of the proximal end (18, 42, 74, 94, 104, 124, 144, 164) by mating with a receiving member which comprises a radially projecting face portion against which the graft (12, 112, 132, 154, 182) is trapped when the engagement portion (24, 50, 78, 92, 102, 122, 142, 170) is moved distally.

11. The apparatus according to any one of the preceding claims, wherein the elongated member (16, 44, 72, 98, 114, 134, 148, 162) comprises a conical-shaped actuator and a conical-shaped opening for receiving the conical-shaped actuator, wherein the graft (12, 112, 132, 154, 182) is mechanically engaged between the conical-shaped actuator and the conical-shaped opening when the conical-shaped actuator is moved towards the conical-shaped opening.

## Patentansprüche

1. Gerät zum Befestigen eines Transplantats an einem Tunnelierer während des subkutanen Implantierens des Transplantats zum vaskulären Zugriff, wobei das Gerät Folgendes umfasst:
ein längliches Organ (16, 44, 72, 98, 114, 134, 148, 162), das dazu ausgelegt ist, um von einem Eintrittspunkt an einer ersten Stelle an der Oberfläche der Haut eines Patienten bis zu einem Austrittspunkt an einer zweiten Stelle an der Oberfläche der Haut des Patienten subkutan tunnelliert zu werden, und ein proximales Ende (18, 42, 74, 94, 104, 124, 144, 164) und ein distales Ende (20, 48, 76, 166) umfasst;
einen Transplantat-Eingriffsabschnitt (24, 50, 78, 92, 102, 122, 142, 170) am proximalen Ende (18, 42, 74, 94, 104, 124, 144, 164) des länglichen Organs (16, 44, 72, 98, 114, 134, 148, 162), wobei der Transplantat-Eingriffsabschnitt (24, 50, 78, 92, 102, 122, 142, 170) Folgendes umfasst: mindestens eine proximal zugewandte Oberfläche, die dazu ausgelegt ist, um die innere Oberfläche eines Transplantats (12, 112, 132, 154, 182) aufzunehmen, so dass sie in der Richtung des distalen Endes (20, 48, 76, 166) geschoben werden kann; und mindestens ein radial vorstehendes Element (26, 80, 96, 106, 130, 152, 178), das dazu ausgelegt ist, um einer Bewegung des Transplantats (12, 112, 132, 154, 182) in der Richtung des proximalen Endes (18, 42, 74, 94, 104, 124, 144, 164) zu widerstehen, nachdem das Transplantat (12, 112, 132, 154, 182) über die proximal zugewandte Oberfläche aufgenommen wurde, und eine abnehmbare Führungsspitze (34) zum Anbringen an dem proximalen Ende (18, 42, 74, 94, 104, 124, 144, 164) des länglichen Organs (16, 44, 72, 98, 114, 134, 148, 162), wobei die abnehmbare Führungsspitze (34) ausgelegt ist, um das Tunnellieren zu erleichtern, und der Eingriffsabschnitt (24, 50, 78, 92, 102, 122, 142, 170) ausgelegt ist, um abnehmbar an der Führungsspitze (34) befestigt zu werden,
**dadurch gekennzeichnet, dass**
das mindestens eine radial vorstehende Element ein Organ (32, 58, 80) umfasst, das radial nach außen vorbelastet ist und mindestens einen Widerhaken (26, 80, 96, 106, 130, 152, 178) umfasst, der radial nach außen vorsteht und konfiguriert ist, um in die innere Oberfläche des Transplantats (12, 112, 132, 154, 182) einzugreifen.

2. Gerät nach Anspruch 1, ferner umfassend eine integrierte Führungsspitze an dem proximalen Ende (18, 42, 74, 94, 104, 124, 144, 164) des länglichen Organs (16, 44, 72, 98, 114, 134, 148, 162), die dazu ausgelegt ist, das Tunnellieren zu erleichtern.

3. Gerät nach einem der vorhergehenden Ansprüche, wobei das nach außen vorbelastete Element (32, 58, 80) auch konfiguriert ist, um zeitweise radial zusammengedrückt zu werden, wobei die proximal zugewandte Oberfläche des Transplantat-Eingriffsabschnitts (24, 50, 78, 92, 102, 122, 142, 170) mindestens teilweise die proximale Oberfläche des nach innen zusammendrückbaren, nach außen vorbelasteten Organs (32, 58, 80) umfasst, die mit dem Eingriffsabschnitt (24, 50, 78, 92, 102, 122, 142, 170) verknüpft ist.

4. Gerät nach einem der vorhergehenden Ansprüche, wobei der Eingriffsabschnitt (24, 50, 78, 92, 102, 122, 142, 170) hakenförmig ist.

5. Gerät nach einem der vorhergehenden Ansprüche, wobei der Eingriffsabschnitt (24, 50, 78, 92, 102, 122, 142, 170) einen im Wesentlichen kreisförmigen Querschnitt aufweist und durchgehende umlaufende Widerhaken umfasst, die konfiguriert sind, um in die innere Oberfläche des Transplantats (12, 112, 132, 154, 182) einzugreifen.

6. Gerät nach einem der vorhergehenden Ansprüche, wobei der Eingriffsabschnitt (24, 50, 78, 92, 102, 122, 142, 170) einen im Wesentlichen kreisförmigen Querschnitt aufweist und teilweise umlaufende Widerhaken umfasst, die konfiguriert sind, um in die innere Oberfläche des Transplantats (12, 112, 132, 154, 182) einzugreifen.

7. Gerät nach einem der vorhergehenden Ansprüche, wobei der Eingriffsabschnitt (24, 50, 78, 92, 102, 122, 142, 170) einen im Wesentlichen rechteckigen Querschnitt aufweist und drehbare Widerhaken umfasst, die konfiguriert sind, um in die innere Oberfläche des Transplantats (12, 112, 132, 154, 182) in einer ersten drehbaren Position einzugreifen und sich auf die proximal zugewandte Oberfläche in einer zweiten drehbaren Position auszurichten.

8. Gerät nach einem der vorhergehenden Ansprüche, wobei ein Teil des Eingriffsabschnitts (24, 50, 78, 92, 102, 122, 142, 170) einen Basisabschnitt und einen Griffabschnitt umfasst, wobei der Griffabschnitt eine Sperre umfasst, die konfiguriert ist, um in eine Wand des Transplantats (12, 112, 132, 154, 182) zwischen dem Griffabschnitt und dem Basisabschnitt einzugreifen.

9. Gerät nach einem der vorhergehenden Ansprüche, wobei
das längliche Organ (16, 44, 72, 98, 114, 134, 148, 162) einen Auslöser und einen biegsamen Griffabschnitt umfasst, der um den Auslöser herum angeordnet ist, wobei der biegsame Griffabschnitt Sperren umfasst und sich ausdehnt, wenn der Auslöser in Richtung auf das Distalende des länglichen Organs (16, 44, 72, 98, 114, 134, 148, 162) bewegt wird, wobei die Haken konfiguriert sind, um in die innere Oberfläche des Transplantats (12, 112, 132, 154, 182) einzugreifen.

10. Gerät nach einem der vorhergehenden Ansprüche, wobei das radial vorstehende Element (26, 80, 96, 106, 130, 152, 178) ausgelegt ist, um einer Bewegung des Transplantats (12, 112, 132, 154, 182) in der Richtung des proximalen Endes (18, 42, 74, 94, 104, 124, 144, 164) zu widerstehen, indem es mit einem Aufnahmeorgan gepaart wird, das einen radial vorstehenden Stirnflächenabschnitt umfasst, an dem das Transplantat (12, 112, 132, 154, 182) festgesetzt ist, wenn der Eingriffsabschnitt (24, 50, 78, 92, 102, 122, 142, 170) distal bewegt wird.

11. Gerät nach einem der vorhergehenden Ansprüche, wobei das längliche Organ (16, 44, 72, 98, 114, 134, 148, 162) einen konisch geformten Auslöser und eine konisch geformte Öffnung zum Aufnehmen des konisch geformten Auslösers umfasst, wobei das Transplantat (12, 112, 132, 154, 182) mechanisch zwischen dem konisch geformten Auslöser und der konisch geformten Öffnung in Eingriff gebracht wird, wenn der konisch geformte Auslöser in Richtung auf die konisch geformte Öffnung bewegt wird.

## Revendications

1. Appareil pour fixer un greffon à un dispositif de tunnelisation pendant l'implantation sous-cutanée du greffon pour un accès vasculaire, ledit appareil comprenant :
un élément allongé (16, 44, 72, 98, 114, 134, 148, 162) qui est conçu pour être tunnelisé en sous-cutané à partir d'un point d'entrée au niveau d'un premier emplacement sur la surface de la peau d'un patient vers un point de sortie au niveau d'un deuxième emplacement sur la surface de la peau du patient et qui comprend une extrémité proximale (18, 42, 74, 94,104, 124, 144, 164) et une extrémité distale (20, 48, 76, 166) ;
une partie d'insertion du greffon (24, 50, 78, 92, 102, 122, 142, 170) au niveau de l'extrémité proximale (18, 42, 74, 94, 104, 124, 144, 164) de l'élément allongé (16, 44, 72, 98, 114, 134, 148, 162), dans lequel la partie d'insertion du greffon (24, 50, 78, 92, 102, 122, 142, 170) comprend : au moins une surface orientée de manière proximale qui est conçue pour recevoir la surface interne d'un greffon (12, 112, 132, 154, 182) de sorte qu'elle puisse coulisser en direction de l'extrémité distale (20, 48, 76, 166) ; et au moins un élément faisant saillie de manière radiale (26, 80, 96, 106, 130, 152, 178) qui est conçu pour résister à un mouvement du greffon (12, 112, 132, 154, 182) en direction de l'extrémité proximale (18, 42, 74, 94, 104, 124, 144, 164), après que le greffon (12, 112, 132, 154, 182) a été reçu sur la surface orientée de manière proximale, et une pointe pilote amovible (34) à fixer à l'extrémité proximale (18, 42, 74, 94, 104, 124, 144, 164) de l'élément allongé (16, 44, 72, 98, 114, 134, 148, 162), dans lequel la pointe pilote amovible (34) est conçue pour faciliter la tunnelisation et la partie d'insertion (24, 50, 78, 92, 102, 122, 142, 170) est conçue pour être fixée de manière amovible à la pointe pilote (34), **caractérisé en ce que**
l'au moins un élément faisant saillie de manière radiale comprend un élément (32, 58, 80) qui est sollicité de manière radiale vers l'extérieur et comprend au moins un ardillon (26, 80, 96, 106, 130, 152, 178) qui fait saillie de manière radiale vers l'extérieur et qui est configuré pour s'insérer dans la surface interne du greffon (12, 112, 132, 154, 182).

2. Appareil selon la revendication 1, comprenant en outre une pointe pilote intégrée au niveau de l'extrémité proximale (18, 42, 74, 94, 104, 124, 144, 164) de l'élément allongé (16, 44, 72, 98, 114, 134, 148, 162), qui est conçue pour faciliter la tunnelisation.

3. Appareil selon l'une quelconque des revendications précédentes, dans lequel l'élément sollicité vers l'extérieur (32, 58, 80) est également configuré pour être temporairement comprimé de manière radiale, dans lequel la surface orientée de manière proximale de la partie d'insertion du greffon (24, 50, 78, 92, 102, 122, 142, 170) comprend au moins partiellement la surface proximale de l'élément sollicité vers l'extérieur et comprimable vers l'intérieur (32, 58, 80) qui est associé à la partie d'insertion (24, 50, 78, 92,102,122,142,170).

4. Appareil selon l'une quelconque des revendications précédentes, dans lequel la partie d'insertion (24, 50, 78, 92, 102, 122, 142, 170) est en forme de crochet.

5. Appareil selon l'une quelconque des revendications précédentes, dans lequel la partie d'insertion (24, 50, 78, 92, 102, 122, 142, 170) présente une section transversale sensiblement circulaire et comprend des ardillons continuellement circonférentiels qui sont configurés pour s'insérer dans la surface interne du greffon (12, 112, 132, 154, 182).

6. Appareil selon l'une quelconque des revendications précédentes, dans lequel la partie d'insertion (24, 50, 78, 92, 102, 122, 142, 170) présente une section transversale sensiblement circulaire et comprend des ardillons partiellement circonférentiels qui sont configurés pour s'insérer dans la surface interne du greffon (12, 112, 132, 154, 182).

7. Appareil selon l'une quelconque des revendications précédentes, dans lequel la partie d'insertion (24, 50, 78, 92, 102, 122, 142, 170) présente une section transversale sensiblement rectangulaire et comprend des ardillons rotatifs qui sont configurés pour s'insérer dans la surface interne du greffon (12, 112, 132, 154, 182) dans une première position rotative et pour s'aligner avec la surface orientée de manière proximale dans une deuxième position rotative.

8. Appareil selon l'une quelconque des revendications précédentes, dans lequel une partie de la partie d'insertion (24, 50, 78, 92, 102, 122, 142, 170) comprend une partie de base et une partie d'agrippement, dans lequel la partie d'agrippement comprend un élément de blocage qui est configuré pour s'insérer dans une paroi du greffon (12, 112, 132, 154, 182) entre la partie d'agrippement et la partie de base.

9. Appareil selon l'une quelconque des revendications précédentes, dans lequel l'élément allongé (16, 44, 72, 98, 114, 134, 148, 162) comprend un actionneur et une partie d'agrippement flexible disposée autour de l'actionneur, dans lequel la partie d'agrippement flexible comprend des éléments de blocage et se déploie lorsque l'actionneur se déplace en direction de l'extrémité distale de l'élément allongé (16, 44, 72, 98, 114, 134, 148, 162), dans lequel les éléments de blocage sont configurés pour s'insérer dans la surface interne du greffon (12, 112, 132, 154, 182).

10. Appareil selon l'une quelconque des revendications précédentes, dans lequel l'élément faisant saillie de manière radiale (26, 80, 96, 106, 130, 152, 178) est conçu pour résister à un mouvement du greffon (12, 112, 132, 154, 182) en direction de l'extrémité proximale (18, 42, 74, 94, 104, 124, 144, 164) par accouplement avec un élément de réception qui comprend une partie de face faisant saillie de manière radiale contre laquelle le greffon (12, 112, 132, 154, 182) est retenu lorsque la partie d'insertion (24, 50, 78, 92, 102, 122, 142, 170) se déplace en direction distale.

11. Appareil selon l'une quelconque des revendications précédentes, dans lequel l'élément allongé (16, 44, 72, 98, 114, 134, 148, 162) comprend un actionneur de forme conique et une ouverture de forme conique pour recevoir l'actionneur de forme conique, dans lequel le greffon (12, 112, 132, 154, 182) est inséré mécaniquement entre l'actionneur de forme conique et l'ouverture de forme conique lorsque l'actionneur de forme conique se déplace en direction de l'ouverture de forme conique.
